(19) Europäisches Patentamt
European Patent Office
Office européen des brevets

(11) Numéro de publication : 0 357 484 B1

(12) # FASCICULE DE BREVET EUROPEEN

(45) Date de publication du fascicule du brevet : **15.07.92 Bulletin 92/29**

(51) Int. Cl.$^5$ : **A61K 7/06**

(21) Numéro de dépôt : **89402280.5**

(22) Date de dépôt : **11.08.89**

(54) **Composition pour induire et stimuler la croissance des cheveux et/ou freiner leur chute, à base d'alkyl-polyglycoside et d'un dérivé de pyrimidine.**

(30) Priorité : **25.08.88 LU 87323**

(43) Date de publication de la demande : **07.03.90 Bulletin 90/10**

(45) Mention de la délivrance du brevet : **15.07.92 Bulletin 92/29**

(84) Etats contractants désignés : **AT BE CH DE ES FR GB IT LI NL SE**

(56) Documents cités :
**DE-A- 3 615 396**
**FR-A- 2 602 421**
**GB-A- 2 128 627**

(73) Titulaire : **L'OREAL**
**14, Rue Royale**
**F-75008 Paris (FR)**

(72) Inventeur : **Grollier, Jean-François**
**16bis Boulevard Morland**
**F-75004 Paris (FR)**

(74) Mandataire : **Casalonga, Alain et al**
**BUREAU D.A. CASALONGA - JOSSE**
**Morassistrasse 8**
**W-8000 München 5 (DE)**

EP 0 357 484 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet une composition pour induire et stimuler la croissance des cheveux et/ou freiner leur chute, contenant, en association, au moins un dérivé de pyrimidine et au moins un agent tensio-actif de la famille des alkylpolyglycosides.

L'homme a un capital de 100.000 à 150.000 cheveux et il est normal de perdre quotidiennement 50 à 100 cheveux. La maintenance de ce capital résulte essentiellement du fait que la vie d'un cheveu est soumise à un cycle pilaire au cours duquel le cheveu se forme, croît et tombe, avant d'être remplacé par un nouveau cheveu qui apparaît dans le même follicule.

On observe au cours d'un cycle pilaire successivement trois phases, à savoir : la phase anagène, la phase catagène et la phase télogène.

Au cours de la première phase, dite anagène, le cheveu passe par une période de croissance active associée à une intense activité métabolique au niveau du bulbe.

La deuxième phase, dite catagène, est transitoire, et elle est marquée par un ralentissement des activités mitotiques. Au cours de cette phase, le cheveu subit une involution, le follicule s'atrophie et son implantation dermique apparaît de plus en plus haute.

La phase terminale, dite télogène, correspond à une période de repos du follicule et le cheveu finit par tomber, poussé par un cheveu naissant en phase anagène.

Ce processus de renouvellement physique permanent subit une évolution naturelle au cours du vieillissement, les cheveux deviennent plus fins, leurs cycles plus courts.

L'alopécie survient lorsque ce processus de renouvellement physique est accéléré ou perturbé, c'est-à-dire que les phases de croissance sont raccourcies, le passage des cheveux en phase télogène est plus précoce et les cheveux tombent en plus grand nombre. Les cycles de croissance successifs aboutissent à des cheveux de plus en plus fins et de plus en plus court, se transformant peu à peu en un duvet non pigmenté. Ce phénomène peut conduire à la calvitie.

Le cycle pilaire est tributaire de nombreux facteurs pouvant entraîner une alopécie plus ou moins prononcée. Parmi ces facteurs, on peut citer les facteurs alimentaires, endocriniens, nerveux, etc.

On détermine la variation de ces différentes catégories de cheveux grâce au trichogramme et en particulier au phototrichogramme.

On recherche depuis de nombreuses années dans l'industrie cosmétique ou pharmaceutique des compositions permettant de supprimer ou de réduire l'alopécie et notamment d'induire ou de stimuler la croissance des cheveux ou de diminuer leur chute.

Dans cette optique, on a déjà proposé des composés tels que l'amino-6 dihydro-1,2 hydroxy-1 imino-2 pipéridino-4 pyrimidine et ses dérivés. De tels composés sont décrits plus particulièrement dans le brevet US-A-4. 139.619.

Pour augmenter l'efficacité des compositions contenant les dérivés pyrimidiques, il s'est avéré intéressant de rechercher une composition bien tolérée contenant le(s) dérivé(s) de pyrimidine qui soit susceptible de rester en contact avec le scalp sans être rincée pendant une durée prolongée afin de favoriser la pénétration de la substance active dans la couche cornée.

Dans cette optique, la demanderesse vient de découvrir qu'en utilisant en association avec les dérivés de pyrimidine, un alkylpolyglycoside, il était possible d'améliorer l'efficacité de la composition dans le traitement de l'induction et de la stimulation de la croissance des cheveux et du freinage de leur chute.

L'association de ces deux composés permet d'obtenir, notamment, par rapport à celles antérieurement connues, des compositions plus efficaces pouvant être utilisées de façon particulièrement aisée et appropriée pour l'application des principes actifs.

L'utilisation d'un alkylpolyglycoside en association avec le dérivé de pyrimidine permet en outre une élimination remarquablement facile de la composition par simple rinçage.

La demanderesse a par ailleurs constaté que la composition présentait une excellente stabilité au stockage.

La composition conforme à l'invention est par ailleurs particulièrement appropriée sur le plan cosmétique et ne provoque pas d'irritation du cuir chevelu, même après un contact prolongé, sans rinçage.

La présente invention a donc pour objet une nouvelle composition pour induire et stimuler la croissance des cheveux et/ou freiner leur chute, à base de dérivés de pyrimidine et d'un alkylpolyglycoside.

Un autre objet de l'invention est constitué par des compositions pharmaceutiques destinées à une application topique à base de cette association.

L'invention a également pour objet un procédé de traitement cosmétique des cheveux et du cuir chevelu grâce à cette composition.

D'autres objets de l'invention apparaîtront à la lecture de la description et des exemples qui suivent.

La composition conforme à l'invention, pour induire et stimuler la croissance des cheveux et/ou freiner leur chute, est essentiellement caractérisée par le fait qu'elle contient dans un milieu physiologiquement acceptable, au moins un alkylpolyglycoside et au moins un composé répondant à la formule :

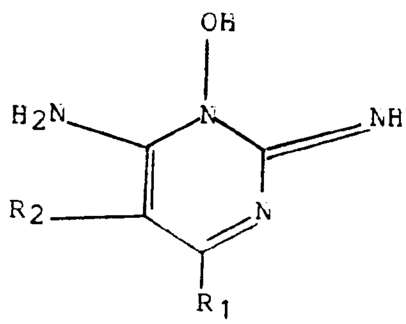

(I)

dans laquelle $R_1$ désigne un groupement

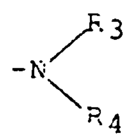

dans lequel :

$R_3$ et $R_4$ peuvent être choisis parmi :

l'hydrogène, un groupement alkyle, ayant de préférence 1 à 4 atomes de carbone, alcényle, alkylaryle ou cycloalkyle inférieur;

$R_3$ et $R_4$ peuvent également former un hétérocycle avec l'atome d'azote auquel ils sont liés, cet hétérocycle étant choisi parmi les groupements aziridinyle, azétidinyle, pyrrolidinyle, pipéridinyle, hexahydroazépinyle, heptaméthylèneimine, octaméthylèneimine, morpholine et alkyle inférieur-4-pipérazidinyle, les groupements hétérocycliques pouvant être substitués sur les atomes de carbone par un à trois groupements alkyle inférieur, hydroxy ou alcoxy;

le groupement $R_2$ est choisi parmi l'hydrogène, un groupement alkyle, alcényle, alcoxyalkyle, cycloalkyle, aryle, alkylaryle, arylalkyle, alkylarylalkyle alcoxyarylalkyle et haloarylalkyle inférieur, ainsi que les sels d'addition d'acides cosmétiquement ou pharmaceutiquement acceptables.

Dans la formule (I), alkyle ou alcoxy désigne de préférence en groupement ayant 1 à 4 atomes de carbone; alcényle désigne de préférence un groupement ayant 2 à 5 atomes de carbone et aryle désigne de préférence phényle.

Les composés de formule (I) plus particulièrement préférés sont choisis parmi les composés dans lesquels $R_2$ désigne hydrogène, et $R_1$ représente un groupement

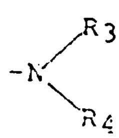

,

dans lequel $R_3$ et $R_4$ forment un cycle pipéridinyle ainsi que leurs sels tel que par exemple le sulfate.

Le composé plus particulièrement préféré est constitué par l'amino-6 dihydro-1,2 hydroxy-1 imino-2 pipéridino-4 pyrimidine ou encore "Minoxidil".

Les alkylpolyglycosides utilisés conformément à l'invention répondent en particulier à la formule (II) :

$$C_nH_{2n+1}\,(C_6H_{10}O_5)_xH \qquad (II)$$

on encore correspondent à la structure développée (III) :

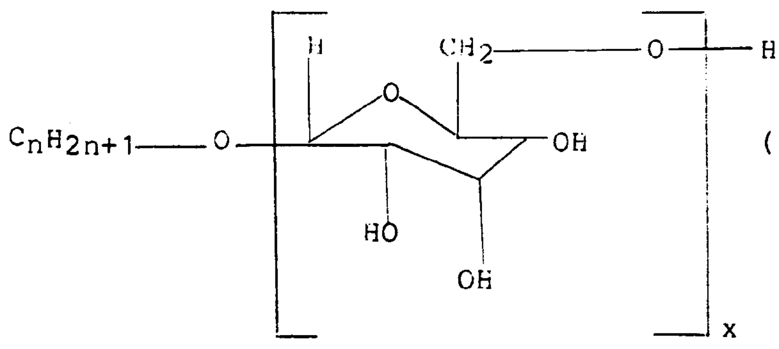

(III)

dans laquelle :

n est un nombre entier compris entre 8 et 15;

x est un nombre entier de 1 à 10.

De tels produits sont vendus par la Société HORIZON CHEMICAL sous la dénomination APG.

Un produit particulièrement préféré est un produit de formule (II), dans laquelle :

n est compris entre 8 et 11; et

x est compris entre 1 et 10.

Un tel produit est en particulier vendu sous la dénomination APG 300 ou APG 500 par la Société HORIZON CHEMICAL sous forme de solution aqueuse à 50% de matière active.

Le dérivé de pyrimidine de formule (I) est utilisé dans les compositions conformes à l'invention, dans une proportion de 0,05 à 6% en poids par rapport au poids total de la composition, et de préférence de 0,1 à 3%, et plus particulièrement de 0,5 à 2%.

L'agent tensio-actif constitué par l'alkylpolyglycoside est utilisé dans des proportions de 1 à 30% en poids par rapport au poids total de la composition et de préférence de 5 à 15%.

Le milieu physiologiquement acceptable est un milieu approprié pour être utilisé en cosmétique ou en pharmacie et peut être constitué par un milieu aqueux ou un mélange d'eau et d'un solvant cosmétiquement ou pharmaceutiquement acceptable, épaissi ou non.

A titre de solvants, on peut utiliser notamment les alcools inférieurs en $C_1$-$C_4$, tels que l'alcool éthylique, l'alcool isopropylique et l'alcool tertiobutylique; les alkylèneglycols comme le propylèneglycol et des alkyléthers de mono- et de dialkylèneglycol, tel que le monoéthyléther d'éthylèneglycol, le monométhyléther de propylèneglycol et le monoéthyléther de diéthylèneglycol.

Les solvants, lorsqu'ils sont utilisés dans le milieu aqueux, sont présents dans des proportions entre 0,5 et 80% en poids par rapport au poids total de la composition.

A titre d'épaississants, on peut utiliser des agents épaississants bien connus dans l'état de la technique parmi lesquels on peut citer plus particulièrement et à titre non limitatif, le diéthanolamide des acides de coprah, les hétéropolysaccharides tels que la gomme de xanthane ou les scléroglucanes, les dérivés de cellulose et les polymères acryliques réticulés ou non, les polyéthylène glycols et leurs dérivés.

Ces épaississants sont utilisés de préférence dans des proportions comprises entre 0,1 et 6% en poids par rapport au poids total de la composition.

Ces compositions peuvent éventuellement renfermer des ingrédients classiquement utilisés dans les compositions cosmétiques ou pharmaceutiques, tels que des agents conservateurs, des agents complexants, des colorants, des agents alcalinisants ou acidifiants, des parfums, des polymères.

Le pH de ces compositions est compris entre 4 et 9 et de préférence entre 7 et 8,5.

Ces compositions peuvent être préssurisées dans les dispositifs aérosols.

Le procédé de traitement pour lutter contre la chute des cheveux et/ou stimuler leur croissance consiste principalement à appliquer sur les zones alopéciques du cuir chevelu et des cheveux d'un individu, une composition telle que définie ci-dessus, à laisser en contact plusieurs heures et éventuellement à rincer.

On peut par exemple appliquer la composition sur les cheveux et le cuir chevelu, le soir, garder la composition toute la nuit au contact et éventuellement effectuer un shampooing le matin ou laver les cheveux à l'aide de cette composition et à laisser à nouveau en contact quelques minutes avant de rincer.

La composition conforme à l'invention s'est révélée particulièrement intéressante lorsqu'elle est appliquée sous forme de lotion capillaire, éventuellement rincée ou même de shampooing.

Le procédé conforme à l'invention présente les caractéristiques d'un traitement thérapeutique de l'alopécie, dans la mesure où la composition agit sur les mécanismes biologiques, notamment sur le cycle pilaire, afin de corriger le dysfonctionnement de celui-ci.

Ce procédé présente également les caractéristiques d'un traitement cosmétiques dans la mesure où il permet d'embellir la chevelure.

Les exemples suivants sont destinés à illustrer l'invention sans pour autant présenter un caractère limitatif.

EXEMPLE 1

On prépare un shampooing de composition suivante :

| | | | |
|---|---|---|---|
| - Alkylpolyglycoside vendu par la Société HORIZON CHEMICAL sous la dénomination APG 300, en solution aqueuse à 50% de matière active (MA) | | 12,0 | g MA |
| - Minoxidil | | 0,5 | g |
| - Sel disodique de l'acide éthylène diaminotétracétique | | 0,2 | g |
| - Conservateur(s) qs | | | |
| - Eau | qsp | 100,0 | g |

On applique ce shampooing sur les cheveux en massant le cuir chevelu.

On laisse poser pendant quelques minutes, puis on rince et on sèche.

EXEMPLE 2

On prépare une lotion de composition suivante :

| | | | |
|---|---|---|---|
| - Alkylpolyglycoside vendu par la Société HORIZON CHEMICAL sous la dénomination APG 300, en solution aqueuse à 50% de matière active (MA) | | 5,0 | g MA |
| - Minoxidil | | 0,5 | g |
| - Alcool éthylique | | 15,0 | g |
| - Eau | qsp | 100,0 | g |

On applique cette lotion sur le cuir chevelu.

On laisse poser environ 30 minutes, puis on rince.

EXEMPLE 3

On prépare une lotion capillaire de composition suivante :

| | | |
|---|---|---|
| - Alkylpolyglycoside vendu par la Société HORIZON CHEMICAL sous la dénomination APG 300, en solution aqueuse à 50% de matière active (MA) | | 5,0 g MA |
| - Minoxidil | | 0,5 g |
| - Monométhyléther de propylèneglycol | | 20,0 g |
| - Conservateur(s) | qs | |
| - Eau | qsp | 100,0 g |

On applique cette lotion sur le cuir chevelu. On laisse poser environ 30 minutes, puis on rince.

EXEMPLE 4

On prépare un shampooing de composition suivante :

| | | |
|---|---|---|
| - Minoxidil | | 0,5 g |
| - Alkylpolyglycoside vendu par la Société HORIZON CHEMICAL sous la dénomination APG 300, en solution aqueuse à 50% de matière active (MA) | | 15,0 g MA |
| - Diéthanolamide des acides de coprah | | 5,0 g |
| - Sel disodique de l'acide éthylène diaminotétracétique | | 0,2 g |
| - Eau | qsp | 100,0 g |

On effectue un shampooing à base de cette composition.
On rince et on sèche.

EXEMPLE 5

On prépare une lotion capillaire de composition suivante :

| | | |
|---|---|---|
| - Alkylpolyglycoside vendu par la Société HORIZON CHEMICAL sous la dénomination APG 350, en solution aqueuse à 50% de matière active (MA) | | 6,0 g MA |
| - Minoxidil | | 0,15 g |
| - Alcool éthylique | | 29,84 g |
| - Agent complexant | qs | |
| - Eau | qsp | 100,0 g |

On applique cette lotion sur les zones alopéciques du cuir chevelu sans faire suivre l'application de rinçage.

EXEMPLE 6

On prépare un shampooing GEL de composition suivante :

| | | |
|---|---|---|
| - Minoxidil | | 0,5 g |
| - Alkylpolyglycoside (APG 500) vendu par la Société HENKEL sous la dénomination MERGITAL CG 60, en solution aqueuse à 60% de matière active (MA) | | 13,0 g MA |
| - Epaississant : dérivé de PEG | | 0,75 g |
| - Conservateurs, complexant | qs | |
| - Eau | qsp | 100,0 g |

EXEMPLE 7

On prépare un shampooing MOUSSE de composition suivante :

| | | |
|---|---|---|
| - Minoxidil | | 0,15 g |
| - Alkylpolyglycoside (APG 500) vendu par la Société HENKEL sous la dénomination MERGITAL CG 60, en solution aqueuse à 60% de matière active (MA) | | 13,0 g MA |
| - Conservateurs, complexant | qs | |
| - Eau | qsp | 100,0 g |

- Conditionnement aérosol :

| | |
|---|---|
| Composition ci-dessus | 95,0 g |

EXEMPLE 8

On prépare un shampooing de composition suivante :

| | | |
|---|---|---|
| - Minoxidil | | 0,25 g |
| - Alkylpolyglycoside vendu par la Société HORIZON CHEMICAL sous la dénomination APG 500, en solution aqueuse à 50% de matière active (MA) | | 10,0 g MA |
| - Agent complexant | qs | |
| - Eau | qsp | 100,0 g |

## Revendications

**Revendications pour les Etats contractants suivants: AT, BE, CA, DE, FR, GB, IT, LI, NL, SE**

1. Composition pour induire et stimuler la croissance des cheveux et/ou freiner leur chute, caractérisée par le fait qu'elle contient dans un milieu physiologiquement acceptable, au moins un alkylpoly-glycoside et au moins un composé répondant à la formule :

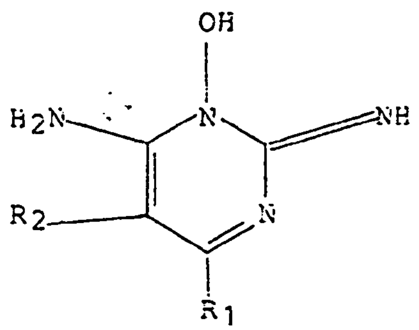

(I)

dans laquelle $R_1$ désigne un groupement

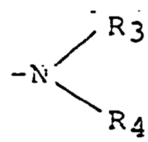

dans lequel :
$R_3$ et $R_4$ peuvent être choisis parmi :
l'hydrogène, un groupement alkyle, alcényle, alkylaryle ou cycloalkyle;
$R_3$ et $R_4$ peuvent également former un hétérocycle avec l'atome d'azote auquel ils sont liés, cet hétérocycle étant choisi parmi les groupements aziridinyle, azétidinyle, pyrrolidinyle, pipéridinyle, hexahydroazépinyle, heptaméthylèneimine, octaméthylèneimine, morpholine et alkyle inférieur-4-pipérazidinyle, les groupements hétérocycliques pouvant être substitués sur les atomes de carbone par un à trois groupements alkyle, hydroxy ou alcoxy;
le groupement $R_2$ est choisi parmi l'hydrogène, un groupement alkyle, alcényle, alcoxyalkyle, cycloalkyle, aryle, alkylaryle, alkylalkyle, alkylarylalkyle, alcoxy-arylalkyle et/ou haloarylalkyle, ainsi que les sels d'addition d'acides physiologiquement acceptables.

2. Composition selon la revendication 1, caractérisée par le fait que le composé (I) est un composé dans lequel $R_2$ désigne l'hydrogène, et $R_1$ représente le groupement :

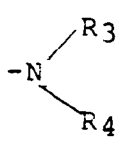

,

dans lequel $R_3$ et $R_4$ forment un cycle pipéridinyle ou l'un de ses sels.

3. Composition selon la revendication 2, caractérisée par le fait que le composé de formule (I) est l'amino-6 dihydro-1,2 hydroxy-1 imino-2 pipéridino-4 pyrimidine ou "Minoxidil".

4. Composition selon l'une quelconque des revendications 1 à 3, caractérisée par le fait que l'alkylpoly-glycoside répond aux formules (II) ou (III) :

$$C_nH_{2n+1}(C_6H_{10}O_5)_xH \qquad (II)$$

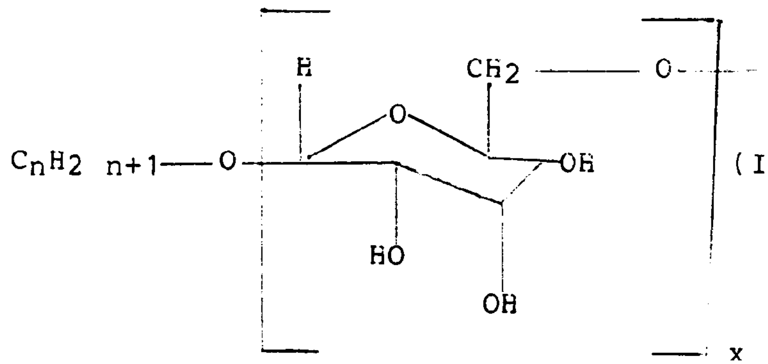

(III)

dans lesquelles n est compris entre 8 et 15 et x est compris entre 1 à 10.

5. Composition selon la revendication 4, caractérisée par le fait que l'alkylpolyglycoside est un composé répondant aux formules (II) ou (III), dans lesquelles n est compris entre 8 et 11 et x est compris entre 1 et 10.

6. Composition selon l'une quelconque des revendications 1 à 5, caractérisée par le fait que le composé de formule (I) est présent dans des proportions comprises entre 0,05 et 6% en poids par rapport un poids total de la composition et de préférence entre 0,1 et 3% en poids.

7. Composition selon l'une quelconque des revendications 1 à 6, caractérisée par le fait que l'alkylpolyglycoside est présent dans des proportions comprises entre 1 et 30% en poids par rapport au poids total de la composition et de préférence entre 5 et 15% en poids.

8. Composition selon l'une quelconque des revendications 1 à 7, caractérisée par le fait que le milieu physiologiquement acceptable est un milieu approprié en cosmétique et en pharmacie et est constitué par de l'eau, un mélange d'eau et d'un solvant cosmétiquement ou pharmaceutiquement acceptable épaissi ou non.

9. Composition selon l'une quelconque des revendications 1 à 8, caractérisée par le fait qu'elle contient un solvant dans des proportions comprises entre 0,5 et 80% en poids par rapport au poids total de la composition.

10. Composition selon l'une quelconque des revendications 1 à 9, caractérisée par le fait qu'elle contient des agents épaississants dans des proportions comprises entre 0,1 et 6% en poids par rapport au poids total de la composition.

11. Procédé de traitement cosmétique des cheveux, caractérisé par le fait que l'on applique sur les cheveux ou le cuir chevelu au moins une composition telle que définie dans l'une quelconque des revendications 1 à 10, qu'on la laisse en contact avec les cheveux et le cuir chevelu et qu'éventuellement on rince.

12. Composition selon l'une quelconque des revendications 1 à 10, pour son application dans le traitement thérapeutique de l'alopécie.

13. Utilisation de la composition selon l'une quelconque des revendications 1 à 10, pour la préparation d'un médicament destiné au traitement thérapeutique de l'alopécie.

**Revendications pour l'Etat contractant suivant: ES**

1. Procédé de préparation d'une composition pour induire et stimuler la croissance des cheveux et/ou freiner leur chute, caractérisé par le fait que l'on incorpore dans un milieu physiologiquement acceptable, au moins un alkylpolyglycoside et au moins un composé répondant à la formule :

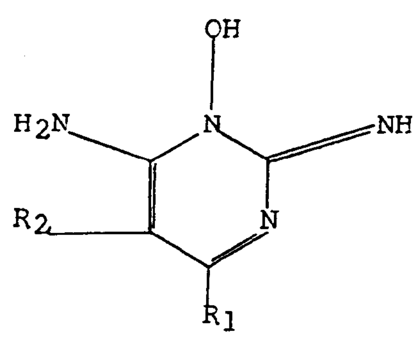

(I)

dans laquelle $R_1$ désigne un groupement

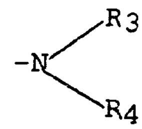

dans lequel :

$R_3$ et $R_4$ peuvent être choisis parmi :

l'hydrogène, un groupement alkyle, alcényle, alkylaryle ou cycloalkyle;

$R_3$ et $R_4$ peuvent également former un hétérocycle avec l'atome d'azote auquel ils sont liés, cet hétérocycle étant choisi parmi les groupements aziridinyle, azétidinyle, pyrrolidinyle, pipéridinyle, hexahydroazépinyle, heptaméthylèneimine, octaméthylèneimine, morpholine et alkyle inférieur-4-pipérazidinyle, les groupements hétérocycliques pouvant être substitués sur les atomes de carbone par un à trois groupements alkyle, hydroxy ou alcoxy;

le groupement $R_2$ est choisi parmi l'hydrogène, un groupement alkyle, alcényle, alcoxyalkyle, cycloalkyle, aryle, alkylaryle, arylalkyle, alkylarylalkyle, alcoxy-arylalkyle et/ou haloarylalkyle, ainsi que les sels d'addition d'acides physiologiquement acceptables.

2. Procédé selon la revendication 1, caractérisé par le fait que le composé (I) est un composé dans lequel $R_2$ désigne l'hydrogène et $R_1$ représente le groupement :

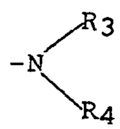 ,

dans lequel $R_3$ et $R_4$ forment un cycle pipéridinyle ou l'un de ses sels.

3. Procédé selon la revendication 2, caractérisé par le fait que le composé de formule (I) est l'amino-6 dihydro-1,2 hydroxy-1 imino-2 pipéridino-4 pyrimidine ou "Minoxidil".

4. Procédé selon l'une quelconque des revendications 1 à 3, caractérisé par le fait que l'alkylpolyglycoside répond aux formules (II) ou (III) :

$$C_nH_{2n+1}(C_6H_{10}O_5)_xH \qquad (II)$$

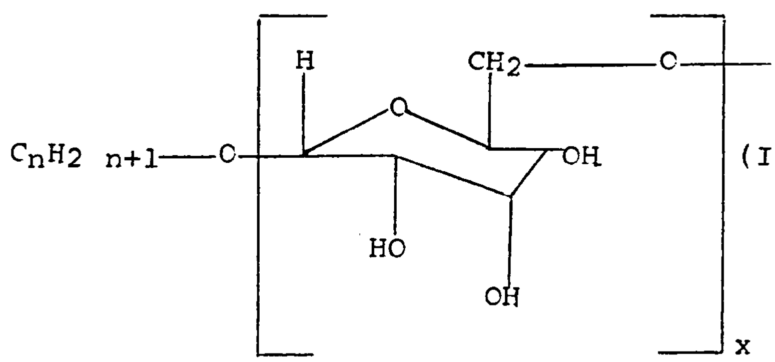 (III)

dans lesquelles n est compris entre 8 et 15 et x est compris entre 1 et 10.

5. Procédé selon la revendication 4, caractérisé par le fait que l'alkylpolyglycoside est un composé répondant aux formules (II) ou (III), dans lesquelles n est compris entre 8 et 11 et x est compris entre 1 et 10.

6. Procédé selon l'une quelconque des revendications 1 à 5, caractérisé par le fait que le composé de formule (I) est présent dans des proportions comprises entre 0,05 et 6% en poids par rapport au poids total de la composition et de préférence entre 0,1 et 3% en poids.

7. Procédé selon l'une quelconque des revendications 1 à 6, caractérisé par le fait que l'alkylpolyglycoside est présent dans des proportions comprises entre 1 et 30% en poids par rapport au poids total de la composition et de préférence entre 5 et 15% en poids.

8. Procédé selon l'une quelconque des revendications 1 à 7, caractérisé par le fait que le milieu physiologiquement acceptable est un milieu approprié en cosmétique et en pharmacie et est constitué par de l'eau, un mélange d'eau et d'un solvant cosmétiquement ou pharmaceutiquement acceptable épaissi ou non.

9. Procédé selon l'une quelconque des revendications 1 à 8, caractérisé par le fait que l'on introduit un solvant dans des proportions comprises entre 0,5 et 80% en poids par rapport au poids total de la composition.

10. Procédé selon l'une quelconque des revendications 1 à 9, caractérisé par le fait que l'on introduit des agents épaississants dans des proportions comprises entre 0,1 et 6% en poids par rapport au poids total de la composition.

11. Procédé de traitement cosmétique des cheveux, caractérisé par le fait que l'on applique sur les cheveux ou le cuir chevelu au moins une composition telle que définie dans l'une quelconque des revendications 1 à 10, qu'on la laisse en contact avec les cheveux et le cuir chevelu et qu'éventuellement on rince.

12. Utilisation de la composition selon l'une quelconque des revendications 1 à 10, pour la préparation d'un médicament destiné au traitement thérapeutique de l'alopécie.

## Patentansprüche

### Patentansprüche für folgende Vertragsstaaten: AT, BE, CH, DE, FR, GB, IT, LI, NL, SE

1. Zusammensetzung zum Induzieren und Stimulieren des Haarwuchses und/oder zum Vermindern des Haarausfalles, dadurch gekennzeichnet, daß sie in einem physiologisch verträglichen Milieu enthält: mindestens ein Alkylpolyglycosid und mindestens eine Verbindung der Formel:

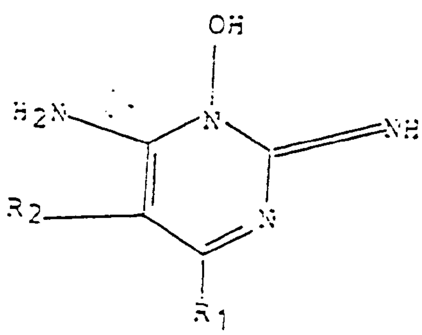

(I)

worin $R_1$ eine

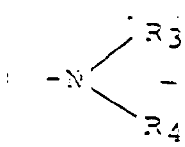

Gruppe darstellt,
worin $R_3$ und $R_4$ unter Wasserstoff, einer Alkyl-, Alkenyl-, Alkylaryl- oder Cycloalkylgruppe ausgewählt sein können, wobei $R_3$ und $R_4$ auch einen Heterozyklus mit dem Stickstoffatom, an das sie gebunden sind, bilden können, wobei dieser Heterozyklus unter Aziridinyl-, Azetidinyl-, Pyrrolidinyl-, Piperidinyl-, Hexahydroazepinyl-, Heptamethylenimin-, Octamethylenimin-, Morpholin- und Niedrigalkyl-4-piperazidinylgruppen ausgewählt ist, wobei die heterozyklischen Gruppen an den Kohlenstoffatomen durch eine bis drei Alkyl-, Hydroxy-oder Alkoxygruppen substituiert sein können, und worin die $R_2$-Gruppe unter Wasserstoff, einer Alkyl-, Alkenyl-, Alkoxyalkyl-, Cycloalkyl-, Aryl-, Alkylaryl-, Arylalkyl-, Alkylarylalkyl-, Alkoxyarylalkyl- und/oder Haloarylalkylgruppe ausgewählt ist, sowie die physiologisch verträglichen Säureadditionssalze davon gibt.

2. Zusammensetzung gemäß Anspruch 1, dadurch gekennzeichnet, daß die Verbindung (I) eine Verbindung ist, worin $R_2$ Wasserstoff und $R_1$ die

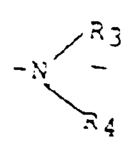

-Gruppe bedeuten, worin $R_3$ und $R_4$ einen Piperidinylring oder eines seiner Salze bilden.

3. Zusammensetzung gemäß Anspruch 2, dadurch gekennzeichnet, daßVerbindung der Formel (I) Amino-6-dihydro-1,2-hydroxi-1-imino-2-piperidino-4-pyrimidin oder "Minoxidil" ist.

4. Zusammensetzung gemäß eines jeden der Ansprüche 1 bis 3, dadurch gekennzeichnet, daß das Alkylpolyglycosid den Formeln (II) oder (III) entspricht:

$$C_nH_{2n+1}(C_6H_{10}O_5)_xH \qquad (II)$$

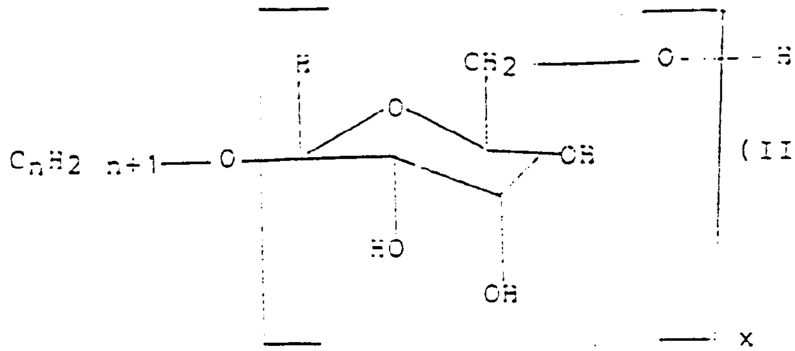

(III)

in denen n eine ganze Zahl von 8 bis 15 und x eine ganze Zahl von 1 bis 10 sind.

5. Zusammensetzung gemäß Anspruch 4, dadurch gekennzeichnet, daß das Alkylpolyglycosid eine Verbindung der Formeln (II) oder (III) ist, in denen n eine ganze Zahl von 8 bis 11 und x eine ganze Zahl von 1 bis 10 sind

6. Zusammensetzung gemäß eines jeden der Ansprüche 1 bis 5, dadurch gekennzeichnet, daß die Verbindung der Formel (I) in Mengen von 0,05 bis 6 Gew.%, bezogen auf Gesamtgewicht der Zusammensetzung, vorzugsweise von 0,1 bis 3 Gew.%, vorliegt.

7. Zusammensetzung gemäß eines jeden der Ansprüche 1 bis 6, dadurch gekennzeichnet, daß das Alkylpolyglycosid in Mengen von 1 bis 30 Gew.%, bezogen auf Gesamtgewicht der Zusammensetzung, vorzugsweise von 5 bis 15 Gew.%, vorliegt.

8. Zusammensetzung eines jeden der Ansprüche 1 bis 7, dadurch gekennzeichnet, daß das physiologisch verträgliche Milieu ein für Kosmetik und Pharmazie geeignetes Milieu ist und aus Wasser, einer Mischung aus Wasser und einem kosmetisch oder pharmazeutisch verträglichen Lösungsmittel, verdickt oder nicht, zusammengesetzt ist.

9. Zusammensetzung gemäß eines jeden der Ansprüche 1 bis 8, dadurch gekennzeichnet, daß sie ein Lösungsmittel in Mengen von 0,5 bis 80 Gew.%, bezogen auf Gesamtgewicht der Zusammensetzung, enthält.

10. Zusammensetzung ,gemäß eines jeden der Ansprüche 1 bis 9, dadurch gekennzeichnet, daß sie Verdickungsmittel in Mengen von 0,1 bis 6 Gew.%, bezogen auf Gesamtgewicht der Zusammensetzung, enthält.

11. Verfahren zur kosmetischen Behandlung der Haare, dadurch gekennzeichnet, daß man auf die Haare oder die behaarte Haut mindestens eine Zusammensetzung ,gemäß eines jeden der Ansprüche 1 bis 10 aufbringt, sie in Kontakt mit den Haaren oder der behaarten Haut beläßt und gegebenenfalls spült.

12. Zusammensetzung gemäß eines jeden der Ansprüche 1 bis 10 zur Verwendung bei der therapeutischen Behandlung der Alopezie.

13. Verwendung der Zusammensetzung gemäß eines jeden der Ansprüche 1 bis 10 zur Herstellung eines Medikaments zur therapeutischen Behandlung der Alopezie.

**Patentansprüche für folgenden Vertragsstaat: ES**

1. Verfahren zur Herstellung einer Zusammensetzung zum Induzieren und Stimulieren des Haarwuchses und/oder zum Vermindern des Haarausfalles, dadurch gekennzeichnet, daß man sie in ein physiologisch verträgliches Milieu gibt: mindestens ein Alkylpolyglycosid und mindestens eine Verbindung der Formel:

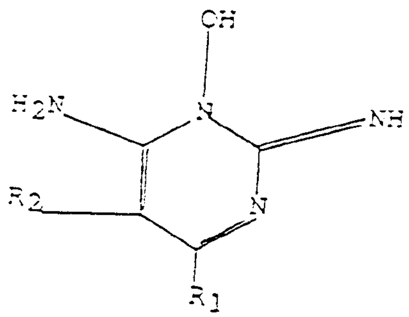

(I)

worin $R_1$ eine

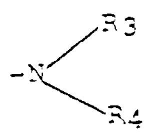

Gruppe darstellt,
worin $R_3$ und $R_4$ unter Wasserstoff,
einer Alkyl-, Alkenyl-, Alkylaryl- oder Cycloalkylgruppe
ausgewählt sein können, wobei $R_3$ und $R_4$ auch einen Heterozyklus mit dem Stickstoffatom, an das sie gebunden sind, bilden können, wobei dieser Heterozyklus unter Aziridinyl-, Azetidinyl-, Pyrrolidinyl-, Piperidinyl-, Hexahydroazepinyl-, Heptamethylenimin-, Octamethylenimin-, Morpholin- und Niedrigalkyl-4-piperazidinylgruppen ausgewählt ist, wobei die heterozyklischen Gruppen an den Kohlenstoffatomen durch eine bis drei Alkyl-, Hydroxy-oder Alkoxygruppen substituiert sein können,
und worin die $R_2$-Gruppe unter Wasserstoff, einer Alkyl-, Alkenyl-, Alkoxyalkyl-, Cycloalkyl-, Aryl-, Alkylaryl-, Arylalkyl-, Alkylarylalkyl-, Alkoxyarylalkyl- und/oder Haloarylalkylgruppe ausgewählt ist, sowie die physiologisch verträglichen Säureadditionssalze davon.

2. Verfahren gemäß Anspruch 1, dadurch gekennzeichnet, daß die Verbindung (I) eine Verbindung ist, worin $R_2$ Wasserstoff und $R_1$ die

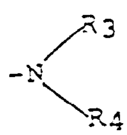

-Gruppe bedeuten, worin $R_3$ und $R_4$ einen Piperidinylring oder eines seiner Salze bilden.

3. Verfahren gemäß Anspruch 2, dadurch gekennzeichnet, daß die Verbindung der Formel (I) Amino-6-dihydro-1,2-hydroxi-1-imino-2-piperidino-4-pyrimidin oder "Minoxidil" ist.

4. Verfahren gemäß eines jeden der Ansprüche 1 bis 3, dadurch gekennzeichnet, daß das Alkylpolyglycosid den Formeln (II) oder (III) entspricht:

$$C_nH_{2n+1}(C_6H_{10}O_5)_xH \qquad (II)$$

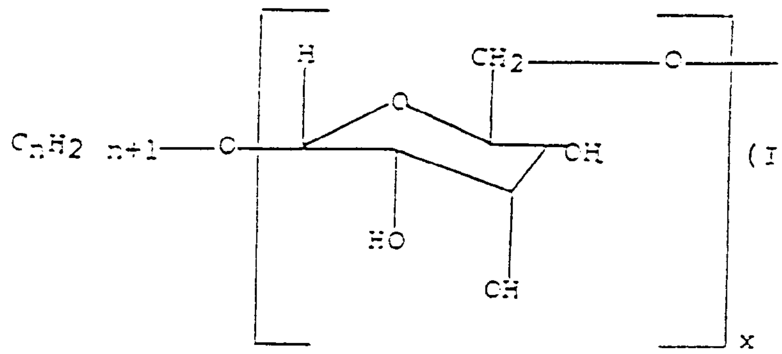

(III)

in denen n eine ganze Zahl von 8 bis 15 und x eine ganze Zahl von 1 bis 10 sind.

5. Verfahren gemäß Anspruch 4, dadurch gekennzeichnet, daß das Alkylpolyglycosid eine Verbindung der Formeln (II) oder (III) ist, in denen n eine ganze Zahl von 8 bis 11 und x eine ganze Zahl von 1 bis 10 sind.

6. Verfahren gemäß eines jeden der Ansprüche 1 bis 5, dadurch gekennzeichnet, daß Verbindung der Formel (I) in Mengen von 0,05 bis 6 Gew.%, bezogen auf Gesamtgewicht der Zusammensetzung, vorzugsweise von 0,1 bis 3 Gew.%, vorliegt.

7. Verfahren gemäß eines jeden der Ansprüche 1 bis 6, dadurch gekennzeichnet, daß das Alkylpolyglycosid in Mengen von 1 bis 30 Gew.%, bezogen auf Gesamtgewicht der Zusammensetzung, vorzugsweise von 5 bis 15 Gew.%, vorliegt.

8. Verfahren eines jeden der Ansprüche 1 bis 7, dadurch gekennzeichnet, daß das physiologisch verträgliche Milieu ein für Kosmetik und Pharmazie geeignetes Milieu ist und aus Wasser, einer Mischung aus Wasser und einem kosmetisch oder pharmazeutisch verträglichen Lösungsmittel, verdickt oder nicht, zusammengesetzt ist.

9. Verfahren gemäß eines jeden der Ansprüche 1 bis 8, dadurch gekennzeichnet, daß sie ein Lösungsmittel in Mengen von 0,5 bis 80 Gew.%, bezogen auf Gesamtgewicht der Zusammensetzung, enthält.

10. Verfahren gemäß eines jeden der Ansprüche 1 bis 9, dadurch gekennzeichnet, daß sie Verdickungsmittel in Mengen von 0,1 bis 6 Gew.%, bezogen auf Gesamtgewicht der Zusammensetzung, enthält.

11. Verfahren zur kosmetischen Behandlung der Haare, dadurch gekennzeichnet, daß man auf die Haare oder die behaarte Haut mindestens eine Zusammensetzung, erhältlich gemäß eines jeden der Ansprüche 1 bis 10, aufbringt, sie in Kontakt mit den Haaren oder der behaarten Haut beläßt und gegebenenfalls spült.

12. Verwendung der Zusammensetzung gemäß eines jeden der Ansprüche 1 bis 10 zur Herstellung eines Medikaments zur therapeutischen Behandlung der Alopezie.

## Claims

### Claims for the following Contracting States: AT, BE, CH, DE, FR, GB, IT, LI, NL, SE

1. Composition for inducing and stimulating hair growth and/or retarding hair loss, characterised in that it contains, in a physiologically acceptable medium, at least one alkylpolyglycoside and at least one compound corresponding to the formula:

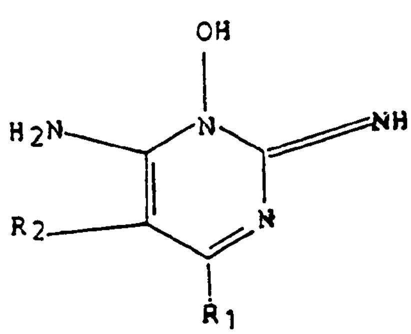

(I)

in which $R_1$ denotes a group

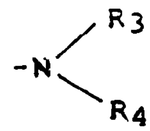

in which:
$R_3$ and $R_4$ may be chosen from:
hydrogen and an alkyl, alkenyl, alkylaryl or cycloalkyl group;
$R_3$ and $R_4$ can also form a heterocycle with the nitrogen atom to which they are linked, this heterocycle being chosen from aziridinyl, azetidinyl, pyrrolidinyl, piperidyl, hexahydroazepinyl, heptamethylenimine, octamethylenimine, morpholine and 4-(lower alkyl)piperazinyl groups, it being possible for the heterocyclic groups to be substituted on the carbon atoms with one to three alkyl, hydroxyl or alkoxy groups;
the group $R_2$ is chosen from hydrogen and an alkyl, alkenyl, alkoxyalkyl, cycloalkyl, aryl, alkylaryl, arylalkyl, alkylarylalkyl, alkoxyarylalkyl and/or haloarylalkyl group; as well as the addition salts with physiologically acceptable acids.

2. Composition according to Claim 1, characterised in that the compound (I) is a compound in which $R_2$ denotes hydrogen and $R_1$ represents the group:

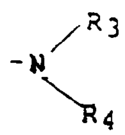

in which $R_3$ and $R_4$ form a piperidyl ring, or one of its salts.

3. Composition according to Claim 2, characterised in that the compound of formula (I) is 6-amino-1,2-dihydro-1-hydroxy-2-imino-4-piperidinopyrimidine or "minoxidil".

4. Composition according to any one of Claims 1 to 3, characterised in that the alkylpolyglycoside corresponds to the formula (II) or (III):

$$C_nH_{2n+1}(C_6H_{10}O_5)_xH \qquad (II)$$

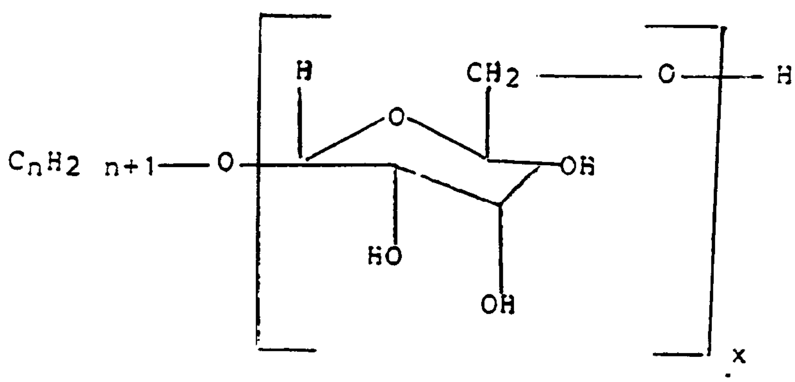

(III)

in which formulae n is between 8 and 15 and x is between 1 and 10.

5. Composition according to Claim 4, characterised in that the alkylpolyglycoside is a compound corresponding to the formula (II) or (III) in which n is between 8 and 11 and x is between 1 and 10.

6. Composition according to any one of Claims 1 to 5, characterised in that the compound of formula (I) is present in proportions of between 0.05 and 6% by weight relative to the total weight of the composition, and preferably between 0.1 and 3% by weight.

7. Composition according to any one of Claims 1 to 6, characterised in that the alkylpolyglycoside is present in proportions of between 1 and 30% by weight relative to the total weight of the composition, and preferably between 5 and 15% by weight.

8. Composition according to any one of Claims 1 to 7, characterised in that the physiologically acceptable medium is a medium suitable for cosmetics and for pharmaceuticals, and consists of water or a mixture of water and a cosmetically or pharmaceutically acceptable solvent, thickened or otherwise.

9. Composition according to any one of Claims 1 to 8, characterised in that it contains a solvent in proportions of between 0.5 and 80% by weight relative to the total weight of the composition.

10. Composition according to any one of Claims 1 to 9, characterised in that it contains thickening agents in proportions of between 0.1 and 6% by weight relative to the total weight of the composition.

11. Process for cosmetic treatment of the hair, characterised in that at least one composition as defined in any one of Claims 1 to 10 is applied on the hair or to the scalp, in that it is left in contact with the hair and the scalp and in that the hair is optionally rinsed.

12. Composition according to any one of Claims 1 to 10, for application in the therapeutic treatment of alopecia.

13. Use of the composition according to any one of Claims 1 to 10 for the preparation of a medicinal product intended for the therapeutic treatment of alopecia.

**Claims for the following Contracting States: ES**

1. Process for preparing a composition for inducing and stimulating hair growth and/or retarding hair loss, characterised in that there are incorporated in a physiologically acceptable medium at least one alkylpolyglycoside and at least one compound corresponding to the formula:

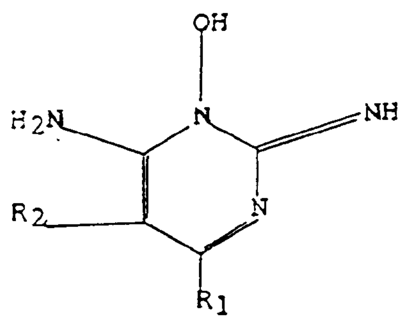

(I)

in which $R_1$ denotes a group

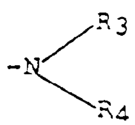

in which:
$R_3$ and $R_4$ may be chosen from:
hydrogen and an alkyl, alkenyl, alkylaryl or cycloalkyl group;
$R_3$ and $R_4$ can also form a heterocycle with the nitrogen atom to which they are linked, this heterocycle being chosen from aziridinyl, azetidinyl, pyrrolidinyl, piperidyl, hexahydroazepinyl, heptamethylenimine, octamethylenimine, morpholine and 4-(lower alkyl)piperazinyl groups, it being possible for the heterocyclic groups to be substituted on the carbon atoms with one to three alkyl, hydroxyl or alkoxy groups;
the group $R_2$ is chosen from hydrogen and an alkyl, alkenyl, alkoxyalkyl, cycloalkyl, aryl, alkylaryl, arylalkyl, alkylarylalkyl, alkoxyarylalkyl and/or haloarylalkyl group; as well as the addition salts with physiologically acceptable acids.

2. Process according to Claim 1, characterised in that the compound (I) is a compound in which $R_2$ denotes hydrogen and $R_1$ represents the group:

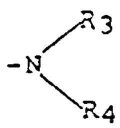

in which $R_3$ and $R_4$ form a piperidyl ring, or one of its salts.

3. Process according to Claim 2, characterised in that the compound of formula (I) is 6-amino-1,2-dihydro-1-hydroxy-2-imino-4-piperidinopyrimidine or "minoxidil".

4. Process according to any one of Claims 1 to 3, characterised in that the alkylpolyglycoside corresponds

to the formula (II) or (III):

$$C_nH_{2n+1}(C_6H_{10}O_5)_xH \qquad (II)$$

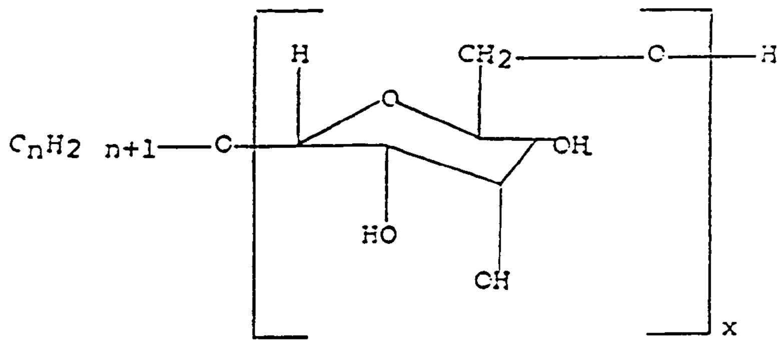

(III)

in which formulae n is between 8 and 15 and x is between 1 and 10.

5. Process according to Claim 4, characterised in that the alkylpolyglycoside is a compound corresponding to the formula (II) or (III) in which n is between 8 and 11 and x is between 1 and 10.

6. Process according to any one of Claims 1 to 5, characterised in that the compound of formula (I) is present in proportions of between 0. 05 and 6% by weight relative to the total weight of the composition, and preferably between 0.1 and 3% by weight.

7. Process according to any one of Claims 1 to 6, characterised in that the alkylpolyglycoside is present in proportions of between 1 and 30% by weight relative to the total weight of the composition, and preferably between 5 and 15% by weight.

8. Process according to any one of Claims 1 to 7, characterised in that the physiologically acceptable medium is a medium suitable for cosmetics and for pharmaceuticals, and consists of water or a mixture of water and a cosmetically or pharmaceutically acceptable solvent, thickened or otherwise.

9. Process according to any one of Claims 1 to 8, characterised in that a solvent is introduced in proportions of between 0.5 and 80% by weight relative to the total weight of the composition.

10. Process according to any one of Claims 1 to 9, characterised in that thickening agents are introduced in proportions of between 0.1 and 6% by weight relative to the total weight of the composition.

11. Process for cosmetic treatment of the hair, characterised in that at least one composition as defined in any one of Claims 1 to 10 is applied on the hair or to the scalp, in that it is left in contact with the hair and the scalp and in that the hair is optionally rinsed.

12. Use of the composition according to any one of Claims 1 to 10 for the preparation of a medicinal product intended for the therapeutic treatment of alopecia.